# EUROPEAN PATENT APPLICATION

(11) **EP 4 803 089 A1**
(43) Date of publication of application: **09.09.2026**
(21) Application number: 24879210.3
(22) Date of filing: 18.10.2024
(51) Int. Cl.: A61K 31/7115, A61K 48/00, C12N 15/113, A61P 27/02

(54) **OLIGONUCLEOTIDES OR ANALOGUES THEREOF FOR THE TREATMENT OF FUCHS ENDOTHELIAL CORNEAL DYSTROPHY**

(30) Priority: 20.10.2023 ES 202330870
(71) Applicant: Arthex Biotech S.L., 46980 Paterna (ES)
(72) Inventor: LLAMUSÍ TROÍSI, María Beatriz, 46980 Valencia (ES); CERRO HERREROS, Estefanía, 46980 Valencia (ES); PIQUERAS LOSILLA, Diego, 46980 Valencia (ES)
(74) Representative: Hoffmann Eitle
(86) International application number: PCT/ES2024/070638
(87) International publication number: WO 2025/083315

(57) **Abstract**

The present invention relates to the use of an oligonucleotide or an oligonucleotide analogue for the manufacture of a drug for the treatment of Fuchs endothelial corneal dystrophy (FECD), wherein the oligonucleotide or oligonucleotide analogue is a microRNA antagonist that down regulates the expression of the human gene MBNL1 and/or MBNL2.

## Description

### TECHNICAL FIELD OF THE INVENTION

The invention relates to the use of oligonucleotides or analogues thereof for therapeutic application against diseases. More specifically, the invention relates to the use of microRNA antagonists, such as antagomiRs, for the treatment of Fuchs endothelial corneal dystrophy

### BACKGROUND OF THE INVENTION

Fuchs endothelial corneal dystrophy (FECD) is a progressive hereditary disease of the posterior cornea characterised by the accelerated loss of corneal endothelial cells (CECs) and the formation of posterior focal excrescences called guttae in the Descemet membrane (DM) (Matthaei *et al*. 2019). It is the most common primary endothelial corneal dystrophy and the main indication of cornea transplant worldwide. It is therefore an extremely common pathology, the prevalence of which is estimated to reach 7.33% in adults over 50 years of age, with higher numbers recorded in the Caucasian population. It is thus estimated that a total of 415 million people will suffer from this disease by 2050 (Aiello *et al*. 2022).

In terms of symptomatology, patients with this pathology suffer from impaired vision due to progressive accelerated loss of CECs and altered DM. It has been described that the loss of CECs in advanced stages leads to impaired corneal endothelial pump function, which limits backflow by controlling the passage of water and solutes to the aqueous humour, maintaining osmotic pressure. This alters the turgor state of the corneal, and accordingly, permeability, leading to the formation of corneal oedema and the formation of painful epithelial blebs. Furthermore, fibroblast-like cell proliferation causing corneal fibrosis is notable. Therefore, although FECD is mainly a corneal endothelial disease, secondary changes may affect all corneal layers. This includes stromal and epithelial layers, as well as corneal nerves.

The genetic basis of FECD is complex and heterogenous with more than 15 genes harbouring associated mutations and/or polymorphisms. Proteins encoded by these genes cover a wide range of endothelial functions, but also DNA repair or transcription regulation. The expansion of non-coding CTG trinucleotide repeats (TNRs) within the third intron (locus CTG18.1) of the transcription factor 4 (TCF4) gene represents the strongest genetic association with FECD (Wieben *et al*. 2017), with a prevalence of 62.1-79% in patients of European descent (Zhang, McGhee, and Patel 2019). Other less common mutations have been found in genes COL8A2, DMPK, SLC4A11, ZEB1/TCF8, and ATP1B1. In turn, SLC4A11 and ATP1B1 are part of the corneal endothelial pump, so they are closely related to the onset of corneal oedema.

The expansion of CTG TNRs in the TCF4 gene causes poly(CUG)n RNA accumulation in CECs that can be visualised as RNA foci. Poly(CUG)n RNA co-localises with the Muscleblind-1 and Mucleblind-2 (MBNL1/2) splicing factors, sequestering them and reducing their functions. MBNL1 is involved in various splicing processes, and its sequestration in the RNA foci causes missplicing or altered transcript processing, giving rise to an incorrect splicing of transcripts. In that sense, altered post-transcriptional processing of genes encoding proteins involved in cytoskeleton protein binding or cell adhesion have been described. As a result, some of the more relevant genes with an altered splicing include NUMA1 (involved in apoptosis), PPFIBP1 (focal adhesion regulation), MBNL1 and MBNL2 (alternative splicing), ADD3 (endothelial junction stabilisation), SYNE1 and ITGA6 (cell adhesion regulation), VEGFA (closely related to the tight junction protein claudin-1), FGFR1 and 2 (involved in the induction of epithelial-mesenchymal transition (EMT) and in the function of fibroblast growth factor receptors 1 and 2), PPHLN1 (formation of the endothelial barrier function) (Du *et al*. 2015; Wieben *et al*. 2017). All this leads to dystrophy in corneal endothelial cells, the loss of corneal endothelial pump function, and the formation of corneal oedema in FECD.

With respect to the current treatment of FECD, there are two palliative drugs approved for the treatment of FECD, anti-oedema eyedrops and ointment, that treat the symptomatology of the disease in an ad hoc manner, with cornea transplant being the only effective treatment. However, this is a highly invasive procedure and requires a cornea donor; therefore, there is a need to find an effective drug therapy that lasts over time.

### DESCRIPTION OF THE FIGURES

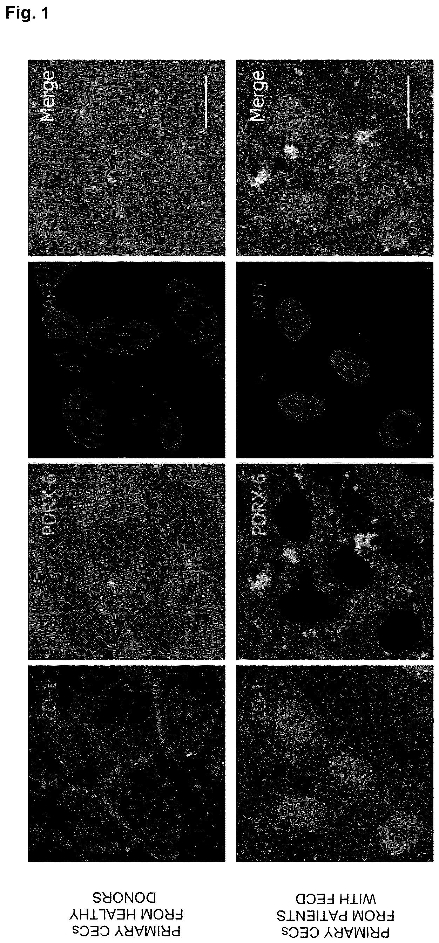

**Figure 1****: Characterisation of the primary corneal endothelial cell model of FECD patients with endothelial markers.** A comparison of ZO-1 and PDRX-6 staining, as endothelial markers, is observed in corneal endothelial cells from healthy donors (top) and FECD patients (bottom). Scale bar = 10 µm.

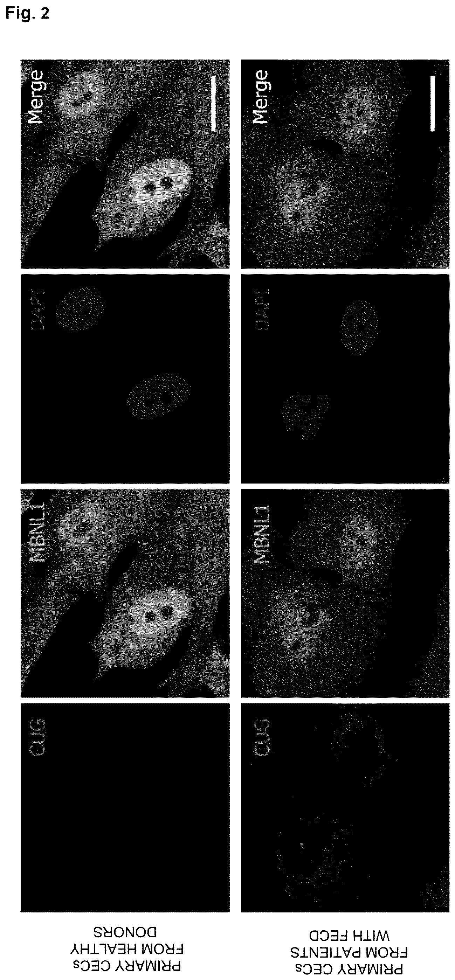

**Figure 2****. Characterisation of the primary corneal endothelial cell model of FECD patients with molecular markers.** A comparison of CUG foci and MBNL1 staining is observed in corneal endothelial cells from healthy donors (top) and FECD patients (bottom). Scale bar = 10 µm.

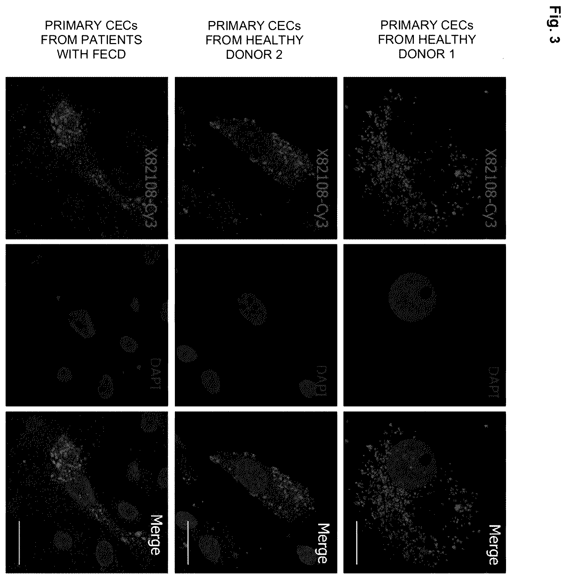

**Figure 3****. Demonstration of the entry of X82108 into primary corneal endothelial cells.** Capacity of X82108 to enter, by means of conjugation with Cy3, into primary corneal endothelial cells from healthy donors and FECD patients was observed. Scale bars = 20 µm

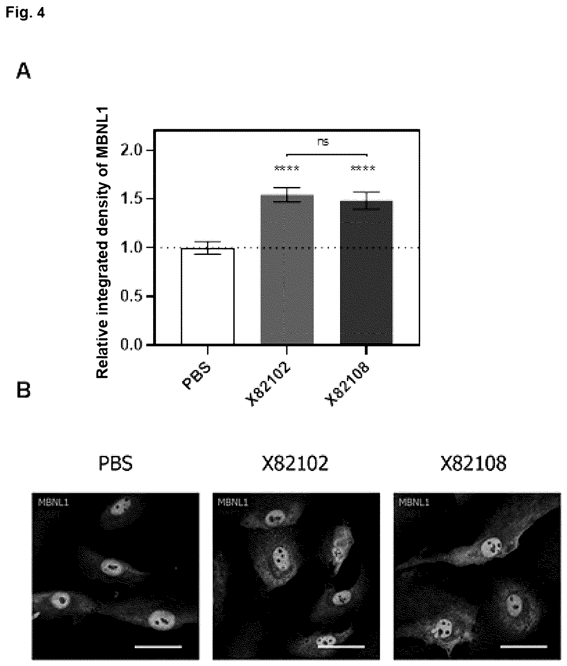

**Figure 4****. X82102 and X82108 increase MBNL1 in primary corneal endothelial cells from healthy donors.** (A) Quantification of the relative integrated density of MBNL1 in primary CECs from healthy donors. Increases in MBNL1 are observed both with X82102 and with X82108, without significant differences between both. Results are expressed as mean with significance **** p<0.0001. (B) Images of MBNL1 IF staining in primary CECs from healthy donors. Scale bars = 20 µm.

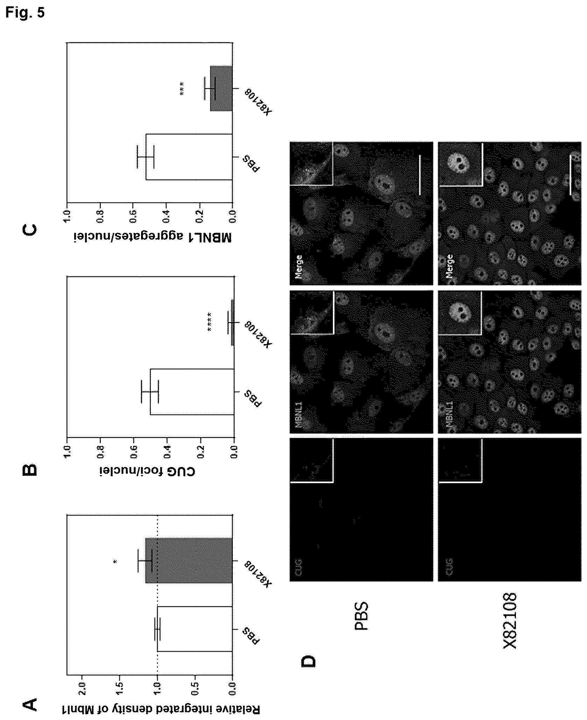

**Figure 5****. X82108 rescues molecular markers in primary corneal endothelial cells from FECD patients.** X82108 increases the integrated density of MBNL1 and reduces both RNA foci and nuclear MBNL1 aggregates. (A) Quantification of the relative integrated density of MBNL1 in primary CECs from FECD patients. (B) Quantification of the number of nuclear RNA foci normalised to the number of total nuclei. (C) Quantification of the number of nuclear MBNL1 aggregates normalised to the number of total nuclei. Results are expressed as mean with significance **** p<0.0001. (D) Images of MBNL1 and RNA foci IF and FISH in primary CECs from FECD patients. Scale bars = 20 µm.

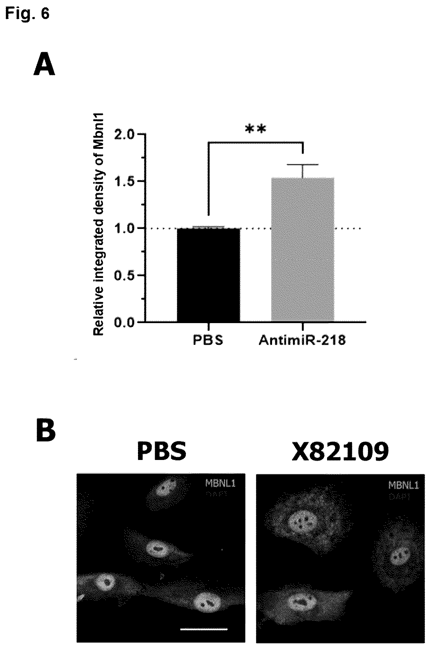

**Figure 6****: Oligonucleotide X82109 increases MBNL1 expression in primary corneal endothelial cells from healthy donors**. (A) Quantification of the relative integrated density of MBNL1 in primary corneal endothelial cells. A significant increase of MBNL1 is observed after the inhibition of miR-218 with X35004. Results are presented as mean ± SEM. ****p<0.0001. (B) Immunofluorescence images of MBNL1 in primary corneal endothelial cells. Scale bar = 20 µm.

### DESCRIPTION OF THE INVENTION

### General Definitions

It should be noted that, as used herein, the singular forms "a", "an", and "the" include plural references, unless the context clearly indicates otherwise. Furthermore, unless indicated otherwise, the term "at least" preceding a series of elements is to be understood as referring to each element of the series. Those skilled in the art will recognise or be capable of determining, based only on routine experimentation, many equivalents to the specific embodiments of the invention described herein. Such equivalents are intended to be encompassed in the present invention.

As used herein, the conjunctive term "and/or" between multiple listed elements is understood to encompass both the individual options and the combined options. For example, when two elements are joined by "and/or", a first option refers to the applicability of the first element without the second element. A second option refers to the applicability of the second element without the first element. A third option refers to the applicability of the first and second elements together. Any of these options is understood within the meaning, and therefore satisfies the requirement of the term "and/or" as used herein. It is also understood that the simultaneous applicability of more than one of the options is comprised in the meaning, and therefore satisfies the requirement of the term "and/or".

Throughout this specification and the following claims, unless the context requires otherwise, the word "comprise" and variations, such as "comprises" and "comprising", shall be understood as involving the inclusion of an integer or a step or a group of integers or steps, but not the exclusion of any other integer or step or group of integers or steps. When used herein, the term "comprising" can be replaced with the term "containing" or "including" or, at times, with the term "having". Any of the mentioned terms (comprising, containing, including, having), as long as it is used herein in the context of an aspect or an embodiment of the present invention, can be replaced with the term "consisting of", although this is less preferred.

When used herein, "consisting of" excludes any element, step, or component not specified in the element of the claim.

A nucleotide "consisting essentially of" a nucleotide is a nucleotide having considerably the same nucleobase sequence as the specified nucleotide.

A nucleotide having "essentially the same nucleotide sequence" as a nucleotide normally has more than 90% nucleobase identity with that nucleotide.

"Nucleic acids", "nucleic acid molecules", "oligonucleotide", and "polynucleotide" are used interchangeably and refer to the polymeric form of phosphate ester of ribonucleosides (adenosine, guanosine, uridine, or cytidine; "RNA molecules") or deoxyribonucleosides (deoxyadenosine, deoxyguanosine, deoxythymidine, or deoxycytidine; "DNA molecules"), or to any phosphoester analogue thereof, such as phosphorothioates and thioesters, in the form of either a single-stranded or a double-stranded helix. The term nucleic acid molecule, and particularly DNA or RNA molecule, refers only to the primary and secondary structures of the molecule and is not limited to any particular tertiary form. Therefore, this term includes the double-stranded DNA found, among others, in linear or circular DNA molecules (for example, restriction fragments), plasmids, supercoiled DNA, and chromosomes.

### Detailed Description

MicroRNAs (commonly abbreviated as miRNAs) are non-coding endogenous RNAs with an approximate length of 15-22 nucleotides that act post-transcriptionally and exert their regulatory effects mainly by means of binding to the 3' UTR region of the target mRNA, resulting in mRNA deadenylation, and thereby causing reduced or supressed translation or, on rare occasions, mRNA cleavage. This last effect, mRNA cleavage, may occur when an mRNA and an miRNA that binds to it fully complement one another, allowing the action of a member of the argonaut protein family, specifically Ago2, which is capable of cleaving the mRNA and causing its direct degradation.

Therefore, the present inventors propose a therapeutic approach for the treatment and/or prevention of Fuchs endothelial corneal dystrophy (FECD) which consists of modulating endogenous MBNL proteins, causing the sequestration of one or more of the miRNAs that act negatively on their expression, thus giving rise to an upregulation and, accordingly, to increased levels of endogenous MBNL proteins. In other words, the aim is to increase endogenous MBNL protein by means of silencing or reducing the repressor activity of specific miRNAs involved in the inhibition of the expression of said protein.

Therefore, a **first aspect** of this invention relates to one or more oligonucleotides or oligonucleotide analogues, also referred to as oligonucleotides of the present invention, which are the inhibitors, silencers, or blockers of an endogenous miRNA. Preferably, the endogenous miRNA is an miRNA that down regulates the expression of the human gene MBNL1 and/or MBNL2. In a preferred embodiment, the endogenous miRNA that down regulates the expression of the human gene MBNL1 and/or MBNL2 is human microRNA-218-5p or human microRNA-23b-3p.

As used herein, inhibitors, silencers, or blockers of an miRNA refer to oligonucleotides which are capable of reducing the endogenous activity of said miRNA. Since the literature related to similar strategies tends to refer to "antagonism", these three terms are encompassed under miRNA "antagonist". Given that the present invention focuses on reducing the activity of miRNAs that repress the expression of certain genes, it is said repressor capacity that will be reduced by the presence of the inhibitors, silencers, or blockers of the miRNAs: their antagonists. Although, strictly speaking, the term "silencing" may be interpreted as the absolute suppression of said activity, given that the difference between the occurrence of said suppression or a non-absolute reduction of repressor activity may depend on the compound concentration used, the four terms (inhibitors, silencers, blockers, or antagonists) are used as synonyms herein, where it is sufficient for a compound to give rise to a reduced miRNA repressor activity for it to be considered an inhibitor, silencer, blocker or, in short, an antagonist of the miRNA. Similarly, the effect caused by an inhibitor, silencer, or blocker is referred to as miRNA inhibition, silencing, or blocking in different parts of the specification, where it is understood that any of those three terms entails and means an antagonism of the action thereof. In a preferred embodiment, the oligonucleotides of the present invention or their analogues are capable of inhibiting endogenous miRNA, preferably human microRNA-218-5p or human microRNA-23b-3p, by at least 10%, 20%, 30%, 40%, 50%, 60%, 70%, 80%, 90%, or 100%, compared to a control oligonucleotide that does not inhibit said endogenous miRNAs. Preferably, the inhibition of endogenous miRNA, preferably human microRNA-218-5p or human microRNA-23b-3p, is a statistically significant inhibition compared to cells or tissues not treated with said oligonucleotides or analogues, wherein the statistical comparison is preferably performed using a Student's t-test.

In other words, in a preferred embodiment, the oligonucleotides of the present invention or their analogues are capable of increasing, in a treated cell, the expression of MBNL proteins (MBNL1 and/or MBNL2) by at least 10%, 20%, 30%, 40%, 50%, 60%, 70%, 80%, 90%, or 100%, compared to a control oligonucleotide, wherein said control oligonucleotide is not capable of binding to, and therefore inhibiting or antagonising, human microRNA-218-5p or microRNA-23b-3p. Preferably, the increase in endogenous levels of MBNL proteins is a statistically significant increase compared to cells or tissues not treated with said oligonucleotides or analogues, wherein the statistical comparison is preferably performed using a Student's t-test. Preferably, the statistically significant increase in endogenous levels of MBNL proteins is at least a log fold change of 0.2, 0.3, 0.4, 0.5, 0.6, 0.7, 0.8, 0.9, 1, 2, 3, 4, 5, or more in treated cells with respect to untreated cells.

Preferably, the treated cell is a cell of the human cornea, more preferably of the corneal endothelium, more preferably a cell of the corneal endothelium of a human patient with FECD.

In some specific points, particularly when reference is made to assays in which miRNA sponges are used, the word "depletion" is also used to refer to the effect produced when said sponges are present, since it can be considered that the number of binding sites in said sponges leads to the binding to said sponges of most or almost all the miRNA molecules having sequences complementary to same, with the miRNA molecules available for interacting with other molecules or compounds in the cell where the sponges are present "being depleted".

As used herein, oligonucleotides refer to molecules resulting from the bonding of at most 50 units of monomers, giving rise to the molecule known for short as DNA or RNA, which monomers are made up of a phosphate group, the nucleobases adenine (A), cytosine (C), guanine (G), thymine (T), or uracil (U), and a pentose, preferably ribose or deoxyribose, or a pentose with a hydroxyl group in position 2'. It should be noted that in the oligonucleotides and/or analogues of the present invention, each uracil and thymine base can be optionally and respectively substituted with a thymine or uracil base. This applies to all "T" nucleobases comprised in all the oligonucleotides disclosed in the present invention. Furthermore, as they are commonly used, molecules having the nucleoside inosine among the units thereof are also considered to be included in said definition.

In a preferred embodiment, the oligonucleotides or analogues thereof are DNA molecules, i.e., molecules formed by the bonding of monomers having a deoxyribose, a phosphate group, and a nucleobase (generally A, T, C, G, U, although others such as inosine "I" can be included). In a preferred embodiment, the oligonucleotides or analogues thereof are RNA molecules, i.e., molecules formed by the bonding of monomers having a ribose or a pentose with a hydroxyl group in position 2', a phosphate group, and a nucleobase (generally A, T, C, G, U, although others such as inosine "I" can be included).

In a preferred embodiment, the oligonucleotides or analogues thereof are molecules that combine DNA bases with RNA bases (a DNA-RNA hybrid).

Furthermore, the oligonucleotides can be oligonucleotide analogues, which means that they comprise chemical modifications. Chemical modifications can be added when they are DNA or RNA or both (a DNA-RNA hybrid). Therefore, the term "oligonucleotides" includes both oligonucleotides *per se* and "oligonucleotide analogues". Molecules derived from oligonucleotides that incorporate a chemical modification in at least one of the nucleotide units making up same, whether in the phosphate group, the pentose, and/or at least one of the nucleobases, are considered "oligonucleotide analogues". Modifications consisting of the addition of non-nucleotide groups at the 5' and/or 3' ends are also included. By extension, for the purposes of the present invention and as used herein, the terms "oligonucleotides" and "oligonucleotide analogues" also include miRNA sponges or sponge miRNAs, since it can be considered that the main constituent thereof are oligonucleotide repeats located in tandem, with the particularity that each of said oligonucleotides contains or is itself a binding site for a microRNA of interest.

With respect to the possible chemical modifications included in the oligonucleotide analogues, the term shall apply in the case of one or more of the common modifications known by those skilled in the art of molecular biology, both in the field of basic research and particularly in the search for the therapeutic applications of said molecules. In a preferred embodiment, the chemical modifications introduced in at least one of the nucleotides forming the oligonucleotide analogue are intended to make the nucleotide more resistant to degradation or more bioavailable. Particularly, for the purposes of the invention, those modifications, valid for oligonucleotides, which give rise to nucleotide analogues with increased resistance to hydrolysis are of special interest (and are considered to be included in the modifications that give rise to molecules included in the scope of the invention), and these modifications are generally modifications in the pentose, including: 2'-O-methyl-substituted (2'-methoxy modifications); 2'-O-methoxyethyl-substituted; LNAs (locked nucleic acids); BNAs (bridged nucleic acids); PMOs (nucleic acids where the ribose has been substituted with a morpholino group), or PNAs (peptide nucleic acids); CRNs (conformationally restricted nucleotides). Modifications that give rise to phosphorothioate linkages and cause the linkages between nucleotides to be resistant to degradation by nucleases, so they are commonly introduced in the last 3-5 nucleotides at the 5' or 3' ends of the oligonucleotides to inhibit their degradation by exonucleases, increasing their stability, are also common and likewise considered to be included in the possible modifications that give rise to the oligonucleotide analogues of the invention. Nucleobase cytosine (C) 5' methylation, which seems to increase the stability of the duplexes formed with the target, is also included among chemical modifications that give rise to the oligonucleotide analogues of the invention due to the frequency of its use within the antimiR group. Chemical modifications of the nucleotide analogues will be described in detail below.

Other different chemical modifications that are likewise comprised in the possible modifications that give rise to oligonucleotide analogues are also possible and known. As can be deduced from the definition of "oligonucleotides" and the definition of "oligonucleotide analogues", molecules that can be considered hybrid in nature, in which some units present modifications and others do not, as well as hybrids between nucleic acid analogues or even hybrid molecules in which some of the nucleotide units are ribonucleotides (or analogues thereof) and others are deoxyribonucleotides (nucleotides where the sugar is deoxyribose), as well as the analogues of the latter, i.e., RNA-DNA hybrids and analogues thereof, are also comprised in the definition of oligonucleotide analogues.

For the purposes of the present invention, miRNA inhibitors, blockers, or antagonists of the types known as blockmiRs, antimiRs, and miRNA sponges are considered to be included in the oligonucleotides or oligonucleotide analogues. In a preferred embodiment, the oligonucleotides of the invention or the analogues thereof are blockmiRs or antimiRs.

As used herein, blockmiRs are small RNAs with a special chemistry designed to target the sequence that a specific miRNA detects in a specific messenger RNA (mRNA), so, in principle, each of them should only de-repress the effect of that miRNA on that transcript, with a very specific effect being expected. Therefore, they are designed such that they have a sequence that is complementary to the sequence of a fragment of the sequence of an mRNA that serves as a binding site for an miRNA, such that they usually bind to the 3' end of the untranslated region (UTR) of an mRNA, i.e., in the area in which endogenous miRNAs usually bind.

In contrast, antimiRs (also referred to as antagomiRs) are used to silence endogenous miRNAs. Therefore, oligonucleotides or oligonucleotide analogues which are complementary to a target miRNA, preferably to an miRNA that down regulates the expression of the human gene MBNL1 and/or MBNL2, more preferably human microRNA-218-5p or human microRNA-23b-3p, are referred to as antagomiRs. Therefore, those oligonucleotides or oligonucleotide analogues that bind specifically to specific miRNAs and thus act as miRNA inhibitors/blockers can be considered as antimiRs.

MicroRNA inhibitors/antagonists are often designed starting from a base nucleotide sequence, the nucleobases of which are complementary, at least in part, to the microRNA to be inhibited (in the case of antimiRs and microRNA sponges) or the sequence of the microRNA itself or a sequence complementary to an area of the mRNA to which the microRNA binds (in the case of blockmiRs). As used herein, it is understood that two oligonucleotides are 100% complementary when the nucleobases thereof pair up perfectly with one another. In other words, the 5'-TAGC-3' sequence and the 5'-UAGC-3' sequence would be 100% complementary to the 3'-ATCG-5' sequence. Sequence alignment for comparison can be carried out with algorithms that align the sequences globally or locally, or by means of computer applications based on said algorithms. Preferably, the alignment algorithm is a local algorithm, preferably BLASt (accessible, for example, through the page of the National Centre for Biotechnology Information of the United States. http://blast.ncbi.nlm.nih.gov/Blast.cgi).

Therefore, to design antagonist molecules (an oligonucleotide or oligonucleotide analogue), it is important to take into account the existence of sufficient complementarity with the endogenous microRNAs or their targets to which they must bind so as to actually produce the desired inhibition/antagonism/silencing effect. In that sense, examples of the "typical" complementarity between an miRNA and its target being 50% can be taken into account, so it is recommendable for the antagonist oligonucleotide or oligonucleotide analogue to comprise a sequence fragment in which the sequence of the nucleobases is at least 50%, 55%, 60%, 70%, 80%, 85%, 90%, 91%, 92%, 93%, 94%, 95%, 96%, 97%, 98%, 99%, or 99.5%, or 100% identical to the sequence of the nucleobases complementary to the fragment of the endogenous molecule with which it should pair up, i.e., to the sequence of the endogenous microRNA to which it must bind (in the case of antimiRs and the repeat sequence of miRNA sponges) or the sequence of the fragment of the messenger mRNA (in the case of blockmiRs).

Therefore, in the case of blockmiRs, the nucleobase sequence of said blockmiRs is preferably at least 80%, 85%, 90%, 91%, 92%, 93%, 94%, 95%, 96%, 97%, 98%, 99%, or 99.5%, or 100% complementary to the nucleobase sequence of the target of the endogenous microRNA that they are intended to antagonise, preferably of the target of the human microRNAs miR-218-5p and miR-23b-3p.

In the case of antimiRs, the nucleobase sequence of said antimiR is preferably at least 80%, 85%, 90%, 91%, 92%, 93%, 94%, 95%, 96%, 97%, 98%, 99%, or 99.5%, or 100%, identical to the nucleobase sequence complementary to the sequence of the endogenous microRNA that they are intended to antagonise, preferably human microRNAs miR-218-5p and miR-23b-3p. In other words, in the case of antimiRs, particularly the nucleobase sequence of said antimiR is preferably at least 80%, 85%, 90%, 91%, 92%, 93%, 94%, 95%, 96%, 97%, 98%, 99%, or 99.5%, or 100% complementary to the nucleobase sequence of the endogenous microRNA that they are intended to antagonise, preferably human microRNAs miR-218-5p and miR-23b-3p.

For the binding of a microRNA to the corresponding messenger RNA, it is fundamental to have in said mRNA the so-called "seed region", a fragment of about 6-8 nucleotides (usually 7), that is part of the area of the mRNA to which the microRNA binds and has perfect complementarity with a part of the microRNA, usually nucleotides 2 to 8 or 9 thereof, which is also often called a seed region. Although the complementarity between the microRNA and its corresponding mRNA is not perfect in the entire pairing area, the complementarity is indeed perfect in the seed region; therefore, the microRNA may be functional in regulating the expression of the gene corresponding to the mRNA containing it. Therefore, in the case of antimiRs, it is highly desirable for the antagonist oligonucleotide or oligonucleotide analogue/inhibitor to comprise a fragment having a sequence of nucleobases that are 100% complementary to the nucleobases of the seed region of the microRNA intended to be antagonised, preferably an miRNA that down regulates the expression of the human gene MBNL1 and/or MBNL2, more preferably human microRNA-218-5p or human microRNA-23b-3p. Therefore, the antimiRs are preferably 100% complementary to at least one part of the mature miRNA which is their target, i.e., the so-called seed region, to which they bind with great affinity. The target region of human microRNA-218-5p or human microRNA-23b-3p is defined as SEQ ID NO: 3 and 4, respectively.

In a preferred embodiment, the oligonucleotide or oligonucleotide analogue is an inhibitor or antagonist of human microRNA-218-5p and comprises a fragment with a sequence of nucleobases which are 100% complementary to the nucleobases of the seed region of human microRNA-218-5p defined as SEQ ID NO: 3. Preferably, the oligonucleotide or oligonucleotide analogue is an inhibitor or antagonist of human microRNA-218-5p and comprises or consists of SEQ ID NO: 1, or comprises or consists of a fragment with a sequence of nucleobases which are at least 50%, 60%, 70%, 80%, 85%, 90%, 91%, 92%, 93%, 94%, 95%, 96%, 97%, 98%, 99%, or 100% complementary to the nucleobases of SEQ ID NO: 1.

In another preferred embodiment, the oligonucleotide or oligonucleotide analogue is an inhibitor or antagonist of human microRNA-23b-3p and comprises a fragment with a sequence of nucleobases which are 100% complementary to the nucleobases of the seed region of human microRNA-23b-3p defined as SEQ ID NO: 4. Preferably, the oligonucleotide or oligonucleotide analogue is an inhibitor or antagonist of human microRNA-23b-3p and comprises or consists of SEQ ID NO: 2, or comprises or consists of a fragment with a sequence of nucleobases which are at least 50%, 60%, 70%, 80%, 85%, 90%, 91%, 92%, 93%, 94%, 95%, 96%, 97%, 98%, 99%, or 100% complementary to the nucleobases of SEQ ID NO: 2.

Preferably, the oligonucleotide or oligonucleotide analogue is an inhibitor or antagonist of human hsa-miR-218-5p and comprises, consists of, or consists essentially of SEQ ID NO: 5 or a sequence with a sequence identity of at least 50%, 60%, 70%, 80%, 85%, 90%, 91%, 92%, 93%, 94%, 95%, 96%, 97%, 98%, 99%, or 100% with respect to SEQ ID NO: 5.

Preferably, the oligonucleotide or oligonucleotide analogue is an inhibitor or antagonist of human microRNA-23b-3p and comprises, consists of, or consists essentially of SEQ ID NO: 6 or a sequence with a sequence identity of at least 50%, 60%, 70%, 80%, 85%, 90%, 91%, 92%, 93%, 94%, 95%, 96%, 97%, 98%, 99%, or 100% with respect to SEQ ID NO: 6.

In a preferred embodiment, the antimiRs are oligonucleotide analogues having chemical modifications that greatly increase binding with their target and their *in vivo* stability:
Internucleotide linkage modifications: Modifications that give rise to phosphorothioate linkages, which are modifications that affect the phosphate groups which are part of the "backbone" of the polynucleotide chain, giving rise to the introduction of a sulphur atom substituting an oxygen atom of the phosphate group that does not act as a bridge between nucleotides, are considered to be included in the possible modifications that give rise to the oligonucleotide analogues of the present invention. These modifications cause the internucleotide linkages to be resistant to degradation by nucleases, in addition to other desirable pharmacological properties, so they are usually inserted between the last 3-5 nucleotides at the 5' or 3' ends of the oligonucleotides to inhibit degradation by exonucleases, increasing the stability thereof.

In a preferred embodiment, at least one, two, three, four, five, six, seven, eight, nine, ten, eleven, twelve, thirteen, fourteen, fifteen, or more than fifteen of the nucleotides comprised in the oligonucleotide or oligonucleotide analogue are chemically bound by means of a phosphorothioate linkage. Preferably, the oligonucleotide or oligonucleotide analogue comprises a length of between 10 and 25 nucleotides and comprises at least two nucleotides chemically bound by means of a phosphorothioate linkage. In an even more preferred embodiment, the oligonucleotide or oligonucleotide analogue molecule comprises a mixture of phosphorothioate and phosphodiester linkages, wherein at least two nucleotides of said molecule are chemically bound by means of a phosphorothioate linkage and at least two nucleotides of said molecule are bound by means of a phosphodiester linkage (PO). In an additional preferred embodiment, the number of nucleotides which are chemically bound by means of a phosphorothioate linkage is greater than the number of nucleotides which are chemically bound by means of a phosphodiester linkage. In one embodiment, the oligonucleotide and/or oligonucleotide analogue molecule has a length between 13 and 17 nucleotides and comprises at least 7, 8, 9, or 10 nucleotides which are chemically bound by means of a phosphorothioate linkage (PS). In one embodiment, the PS:PO ratio in the oligonucleotide and/or oligonucleotide analogue molecule is 1.2:1, 1.5:1, 1.7:1, 2:1, 2.2:1, 2.5:1, 2.7:1, 3: 1. Preferably, the PS:PO ratio in the oligonucleotide and/or oligonucleotide analogue molecule is between 1.2:1 and 2.7:1, more preferably 1.5:1 or 2.5:1. "PS:PO ratio" refers herein to the number of PS linkages per PO linkage.

In one embodiment, all the nucleotides comprised in the oligonucleotide or oligonucleotide analogue are chemically bound by means of a phosphorothioate linkage. As indicated above, the oligonucleotide or oligonucleotide analogue molecule is preferably a microARN antagonist (i.e., an antimiR).

Pentose (ribose or deoxyribose) modifications: The most widely used sugar modifications are those located in the OH group in position 2'. Among them, the most important in the context of the present invention are 2'fluoro (2'F: introduction of a fluorine atom in position 2' of pentose), 2'-O-methoxyethyl (MOE), or 2'O-methyl modifications (OMe). Therefore, in one embodiment, the oligonucleotide or oligonucleotide analogue, preferably the antimiR, according to the present invention is chemically modified to comprise at least one pentose with one of the following modifications: 2'fluoro (2'F: introduction of a fluorine atom in position 2' of ribose), 2'-O-methoxyethyl (MOE), and/or 2'O-methyl (OMe). In one embodiment, all the nucleotides in the oligonucleotide molecule are nucleotides modified with 2'OME.

Another modification that can be performed on the oligonucleotide or oligonucleotide analogue, preferably the antimiR, of the present invention is the formation of 2'-4' bicyclic modifications. There is a variety of ribose derivatives that block the carbohydrate ring in the endo 3' conformation by means of the formation of bicyclic structures with a bridge between oxygen 2' and position 4'. In one embodiment, the formation of a bridge between oxygen 2' and carbon 4' blocks the ribose in the endo 3' conformation, which leads to a modification called locked nucleic acids or LNAs. The introduction of LAN modifications greatly increases the stability of the hybrids resulting from the miARN which is the target of the antimiR, making them considerably more thermodynamically stable and resistant to degradation, which occurs particularly when said modifications are at the ends of the molecule. In one embodiment, the first nucleotide of the 3' region of the oligonucleotide or oligonucleotide analogue comprises an LNA modification. In another embodiment, the first two nucleotides of the 5' region of the oligonucleotide or oligonucleotide analogue comprise an LNA modification. Additional modifications of the bicyclic nucleotides include bridged nucleic acids, ethyl-bridged nucleic acids (ENAs), constrained-ethyl nucleic acids (cEt), bicyclic structures (bicycle-DNA) and tricyclic structures (tricycle-DNA), and conformationally restricted nucleotides (CRNs).

Additional modifications include the so-called PMO (nucleic acids where the ribose has been substituted with a morpholino group). "Morpholino" is understood as bases bound to a backbone of methylene morpholine rings bound through phosphorodiamidate groups.

Other modifications that may be present in the oligonucleotide or the analogues thereof are the so-called PNAs (peptide nucleic acids: peptide nucleic acids in which the ribose-phosphate group is replaced with an amino acid fraction such that the backbone of the nucleotide analogue is a structure with repeat units of N-(2-aminoethyl)-glycine bound by peptide linkages).

In one embodiment, the oligonucleotide or oligonucleotide analogue, preferably the antimiR, is chemically modified to comprise at least one pentose of the nucleotides forming the antimiR comprising morpholino nucleic acids (PMOs) or peptide nucleic acids (PNAs).

Nucleobase modifications: Nucleobase cytosine (C) 5-methylation, which reduces oligonucleotide analogue detection by the immune system, is also included among the chemical modifications that give rise to the oligonucleotides or analogues thereof, preferably the antimiR, due to its frequent use. Therefore, in a preferred embodiment, at least one, two, three, four, five, or more than five of the nucleotides comprised in the oligonucleotide and/or oligonucleotide analogue, preferably the antimiR, comprises a methylated cytosine. In a preferred embodiment, all the cytosines in the oligonucleotide or oligonucleotide analogue, preferably the antimir, are methylated.

Another possible modification is 2,6-diaminopurine which is capable of forming base pairs with thymidine or uridine with an additional H linkage (3 H linkages instead of 2 H linkages present in the natural A:T base pairs). Therefore, in a preferred embodiment, at least one, two, three, four, five, or more than five of the nucleotides comprised in the oligonucleotide and/or oligonucleotide analogue comprise 2,6-diaminopurine.

As can be deduced from the definition of "oligonucleotides" and the definition of "oligonucleotide analogues", also hybrid molecules, in which some nucleotides present modifications and others do not, as well as hybrids between nucleic acid analogues and peptides, or even hybrid molecules in which some units of the molecule are nucleotides (nucleotides in which the sugar is ribose) and other units are deoxynucleotides (nucleotides in which the sugar is deoxyribose), as well as analogues of the latter, i.e., RNA-DNA hybrids and analogues thereof. Other chemical modifications, which are also comprised in the possible modifications that give rise to oligonucleotide analogues, are possible and known.

Preferably, the oligonucleotide or oligonucleotide analogue is an inhibitor or antagonist of human microRNA-23b-3p and comprises, consists of, or consists essentially of SEQ ID NO: 7 or a sequence with a sequence identity of at least 50%, 60%, 70%, 80%, 85%, 90%, 91%, 92%, 93%, 94%, 95%, 96%, 97%, 98%, 99%, or 100% with respect to SEQ ID NO: 7. In some embodiments, the oligonucleotide or oligonucleotide analogue is an inhibitor or antagonist of human microRNA-23b-3p and comprises, consists of, or consists essentially of SEQ ID NO: 7. It should be noted that SEQ ID NO: 7 includes specific chemical modifications, as defined in the "Sequence Listing" section.

Preferably, the oligonucleotide or oligonucleotide analogue is an inhibitor or antagonist of human microRNA-23b-3p and comprises, consists of, or consists essentially of SEQ ID NO: 13 or a sequence with a sequence identity of at least 50%, 60%, 70%, 80%, 85%, 90%, 91%, 92%, 93%, 94%, 95%, 96%, 97%, 98%, 99%, or 100% with respect to SEQ ID NO: 13. In some embodiments, the oligonucleotide or oligonucleotide analogue is an inhibitor or antagonist of human microRNA-23b-3p and comprises, consists of, or consists essentially of SEQ ID NO: 13. It should be noted that SEQ ID NO: 13. includes specific chemical modifications, as defined in the "Sequence Listing" section.

Preferably, the oligonucleotide or oligonucleotide analogue is an inhibitor or antagonist of human microRNA-218-5p and comprises, consists of, or consists essentially of SEQ ID NO: 10 or a sequence with a sequence identity of at least 50%, 60%, 70%, 80%, 85%, 90%, 91%, 92%, 93%, 94%, 95%, 96%, 97%, 98%, 99%, or 100% with respect to SEQ ID NO: 10. In some embodiments, the oligonucleotide or oligonucleotide analogue is an inhibitor or antagonist of human microRNA-218-5p and comprises, consists of, or consists essentially of SEQ ID NO: 10. It should be noted that SEQ ID NO: 10 includes specific chemical modifications, as defined in the "Sequence Listing" section.

In some embodiments, other non-nucleotide molecules, such as organic compounds, can also be conjugated at the 3' and/or 5' ends of the oligonucleotide and/or oligonucleotide analogue molecule. Therefore, other modifications included in the present invention is the addition of fatty acids, preferably oleic acid, at one of the ends of the oligonucleotide or analogue thereof. Preferably, the fatty acid molecule is added to the 3' and/or 5' ends of said oligonucleotide and/or oligonucleotide analogue molecule. Therefore, in a preferred embodiment, the oligonucleotide or nucleotide analogue is conjugated to an oleic acid at its 3' and/or 5' ends. Preferably, the oligonucleotide or nucleotide analogue described above with SEQ ID NOs: 7, 10, and 13, comprises an oleic acid molecule conjugated at the 3' end of said oligonucleotide or nucleotide analogue.

Said conjugation can be a direct conjugation or by means of a spacer molecule. "Direct conjugation" refers to the preferably covalent bonding between the first nucleotide (if the bonding is at the 5' end) and/or the last nucleotide (if the bonding is at the 3' end) of the oligonucleotide or oligonucleotide analogue and the oleic acid molecule. "Spacer" or "spacer molecule" refers to any molecule or molecules connecting, on one hand, the oligonucleotide or oligonucleotide analogue and, on the other hand, the non-nucleotide molecule, preferably oleic acid. Spacer molecule or molecules can be coupled to the 3' or 5' end of the oligonucleotide or oligonucleotide analogue. Preferably, the spacer molecule is covalently bound to said oligonucleotide. Preferably, the spacer molecule(s) is/are bound, at one end, to an oxygen group in the 3'-terminal phosphate of the oligonucleotide by means of a linkage between a terminal carbon of the spacer and, at the other end, to the carboxy group of oleic acid by means of a linkage between the terminal nitrogen group in the connector which forms an amide linkage.

In a preferred embodiment, the spacer is selected from the group consisting of 3-aminopropyl (NHC3), 5-aminopentyl (NHC5), 6-aminohexyl (NHC6), threoninol, or a derivative thereof. In other embodiments, the spacer may comprise a thiol-modifier C6 S-S (C6SSC6). In an additional embodiment, the spacer may comprise a thiol-modifier C6 S-S (C6SSC6) directly bound to the oligonucleotide, and followed by 3-aminopropyl (NHC3), 6-aminohexyl (NHC6), threoninol, or a derivative thereof.

The spacer can be a linear or branched aliphatic hydrocarbon chain, cyclohexylphenyl, and other aromatic spacers, as well as polar spacers based on one or more ethylene glycol, glycerol, amino acid, peptide, or carbohydrate units. In some cases, the oleic can be covalently bound to amino groups by means of an amide linkage or directly to the nucleobases with an amine linkage, as well as to the phosphate linkage as oleyl phosphate

Preferably, oleic acid is conjugated to the oligonucleotide or oligonucleotide analogue at its 3' end. More preferably, oleic acid is conjugated to the oligonucleotide or oligonucleotide analogue by means of a spacer molecule, preferably NHC6, threoninol, or NHC3.

Preferably, the oligonucleotide or oligonucleotide analogue is conjugated to at least one oleic acid molecule at its 3' and/or 5' ends, and furthermore has one or more chemical modifications.

Preferably, the oligonucleotide or oligonucleotide analogue is an inhibitor or antagonist of human microRNA-23b-3p and comprises, consists of, or consists essentially of SEQ ID NO: 8 or a sequence with a sequence identity of at least 50%, 60%, 70%, 80%, 85%, 90%, 91%, 92%, 93%, 94%, 95%, 96%, 97%, 98%, 99%, or 100% with respect to SEQ ID NO: 8. In some embodiments, the oligonucleotide or oligonucleotide analogue is an inhibitor or antagonist of human microRNA-23b-3p and comprises, consists of, or consists essentially of SEQ ID NO: 8. It should be noted that SEQ ID NO: 8 includes specific chemical modifications, as defined in the "Sequence Listing" section, and furthermore includes conjugating an oleic acid to the 3' of the oligonucleotide, wherein said conjugation can be performed directly or by means of a spacer, which is a molecule that connects the 3' end of the oligonucleotide with the oleic acid. Preferably, the spacer is selected from the group of NHC3, NHC5, NHC6, threoninol, or derivatives thereof.

Preferably, the oligonucleotide or oligonucleotide analogue is an inhibitor or antagonist of human microRNA-23b-3p and comprises, consists of, or consists essentially of SEQ ID NO: 9 or a sequence with a sequence identity of at least 50%, 60%, 70%, 80%, 85%, 90%, 91%, 92%, 93%, 94%, 95%, 96%, 97%, 98%, 99%, or 100% with respect to SEQ ID NO: 9. In some embodiments, the oligonucleotide or oligonucleotide analogue is an inhibitor or antagonist of human microRNA-23b-3p and comprises, consists of, or consists essentially of SEQ ID NO: 9. It should be noted that SEQ ID NO: 9 includes specific chemical modifications, as defined in the "Sequence Listing" section, and furthermore includes conjugating an oleic acid to the 3' of the oligonucleotide, wherein said conjugation is performed through a spacer which is NHC6.

Preferably, the oligonucleotide or oligonucleotide analogue is an inhibitor or antagonist of human microRNA-23b-3p and comprises, consists of, or consists essentially of SEQ ID NO: 14 or a sequence with a sequence identity of at least 50%, 60%, 70%, 80%, 85%, 90%, 91%, 92%, 93%, 94%, 95%, 96%, 97%, 98%, 99%, or 100% with respect to SEQ ID NO: 14. In some embodiments, the oligonucleotide or oligonucleotide analogue is an inhibitor or antagonist of human microRNA-23b-3p and comprises, consists of, or consists essentially of SEQ ID NO: 14. It should be noted that SEQ ID NO: 14 includes specific chemical modifications, as defined in the "Sequence Listing" section, and furthermore includes conjugating an oleic acid to the 3' of the oligonucleotide, wherein said conjugation can be performed directly or by means of a spacer, which is a molecule that connects the 3' end of the oligonucleotide with the oleic acid. Preferably, the spacer is selected from the group of NHC3, NHC5, NHC6, threoninol, or derivatives thereof.

Preferably, the oligonucleotide or oligonucleotide analogue is an inhibitor or antagonist of human microRNA-23b-3p and comprises, consists of, or consists essentially of SEQ ID NO: 15 or a sequence with a sequence identity of at least 50%, 60%, 70%, 80%, 85%, 90%, 91%, 92%, 93%, 94%, 95%, 96%, 97%, 98%, 99%, or 100% with respect to SEQ ID NO: 15. In some embodiments, the oligonucleotide or oligonucleotide analogue is an inhibitor or antagonist of human microRNA-23b-3p and comprises, consists of, or consists essentially of SEQ ID NO: 15. It should be noted that SEQ ID NO: 15 includes specific chemical modifications, as defined in the "Sequence Listing" section, and furthermore includes conjugating an oleic acid to the 3' of the oligonucleotide, wherein said conjugation is performed through a spacer which is NHC6.

Preferably, the oligonucleotide or oligonucleotide analogue is an inhibitor or antagonist of human microRNA-218-5p and comprises, consists of, or consists essentially of SEQ ID NO: 11 or a sequence with a sequence identity of at least 50%, 60%, 70%, 80%, 85%, 90%, 91%, 92%, 93%, 94%, 95%, 96%, 97%, 98%, 99%, or 100% with respect to SEQ ID NO: 11. In some embodiments, the oligonucleotide or oligonucleotide analogue is an inhibitor or antagonist of human microRNA-218-5p and comprises, consists of, or consists essentially of SEQ ID NO: 11. It should be noted that SEQ ID NO: 11 includes specific chemical modifications, as defined in the "Sequence Listing" section, and furthermore includes conjugating an oleic acid to the 3' of the oligonucleotide, wherein said conjugation can be performed directly or by means of a spacer, which is a molecule that connects the 3' end of the oligonucleotide with the oleic acid. Preferably, the spacer is selected from the group of NHC3, NHC5, NHC6, threoninol, or derivatives thereof.

Preferably, the oligonucleotide or oligonucleotide analogue is an inhibitor or antagonist of human microRNA-218-5p and comprises, consists of, or consists essentially of SEQ ID NO: 12 or a sequence with a sequence identity of at least 50%, 60%, 70%, 80%, 85%, 90%, 91%, 92%, 93%, 94%, 95%, 96%, 97%, 98%, 99%, or 100% with respect to SEQ ID NO: 12. In some embodiments, the oligonucleotide or oligonucleotide analogue is an inhibitor or antagonist of human microRNA-218-5p and comprises, consists of, or consists essentially of SEQ ID NO: 12. It should be noted that SEQ ID NO: 12 includes specific chemical modifications, as defined in the "Sequence Listing" section, and furthermore includes conjugating an oleic acid to the 3' of the oligonucleotide, wherein said conjugation is performed through a spacer which is NHC6.

Preferably, the oligonucleotide is an oligonucleotide analogue comprising:
a) one or more chemical modifications in the ribose moiety, phosphate linkage, or both, of at least one of the nucleotides making up same
b) a nucleobase sequence which is 100% complementary to the nucleobase sequence of the seed region of the microRNA of SEQ ID NO: 1 (human microRNA-218-5p) or SEQ ID NO: 2 (human microRNA-23b-3p), and
c) optionally, a conjugated fatty acid at the 5' end and/or the 3' end.

Preferably, the oligonucleotide is an oligonucleotide analogue comprising:
a) one or more chemical modifications in the ribose moiety, phosphate linkage, or both, of at least one of the nucleotides making up same
b) a nucleobase sequence that is at least 80% 85%, 90%, 95%, or 100% complementary to the nucleobase sequence of SEQ ID NO: 2 (human microRNA-23b-3p) or SEQ ID NO: 1 (human microRNA-218-5p), and
c) optionally, a conjugated fatty acid at the 5' end and/or the 3' end.

Preferably, the oligonucleotide is an oligonucleotide analogue comprising:
a) one or more chemical modifications in the ribose moiety, phosphate linkage, or both, of at least one of the nucleotides making up same
b) the nucleobase sequence of SEQ ID NO: 6 or SEQ ID NO: 5, or a sequence that is at least 85% identical to the nucleobase sequence of SEQ ID NO: 6 or SEQ ID NO: 5, respectively, and
c) optionally, a conjugated fatty acid at the 5' end and/or the 3' end.

Preferably, the oligonucleotide is an oligonucleotide analogue comprising:
a) at least one, preferably all, of the following chemical modifications:
   - greater number of phosphorothioate linkages than phosphodiester linkages,
   - the last nucleotide located at the 5' end is an LNA modified oligonucleotide,
   - the last nucleotide located at the 3' end is a modified oligonucleotide of,
b) the nucleobase sequence of SEQ ID NO: 6 or SEQ ID NO: 5, or a sequence at least 85%, 90%, 95%, or 100% identical to the nucleobase sequence of SEQ ID NO: 6 or SEQ ID NO: 5, respectively, and
c) optionally, a conjugated fatty acid at the 5' end and/or the 3' end.

Preferably, the oligonucleotide is an oligonucleotide analogue comprising:
a) at least one, preferably all, of the following chemical modifications:
   - greater number of phosphorothioate linkages than phosphodiester linkages,
   - the two nucleotides located at the 5' end are LNA modified oligonucleotides,
   - the two nucleotides located at the 3' end are LNA modified oligonucleotides,
b) the nucleobase sequence of SEQ ID NO: 6 or SEQ ID NO: 5, or a sequence at least 85%, 90%, 95%, or 100% identical to the nucleobase sequence of SEQ ID NO: 6 or SEQ ID NO: 5, respectively, and
c) optionally, a conjugated fatty acid at the 5' end and/or the 3' end.

The first aspect of the present invention also comprises compositions which comprise at least one of these oligonucleotides or oligonucleotide analogues targeting human microRNA-218-5p or human microRNA-23b-3p or mixtures thereof, or generally any oligonucleotide or oligonucleotide analogue that is an inhibitor of one of said microRNAs or of another microRNA that down regulates the expression of the human gene MBNL1 and/or MBNL2, including compositions which also comprise pharmaceutically acceptable vehicles and/or excipients. Furthermore, given the direct relationship between the expression vectors expressing miRNA sponges, or even the precursors of mature microRNAs that finally present a repressor effect, it is considered that the scope of the present invention also comprises a composition comprising an expression vector of at least one oligonucleotide or oligonucleotide analogue, particularly the vectors comprising the coding sequence of a microRNA sponge which comprises multiple sites located in tandem complementary to human microRNA-218-5p or human microRNA-23b-3p or a mixture of multiples binding sites located in tandem complementary to each of them.

In a possible embodiment, the pharmaceutical composition comprises an effective dose of the inhibitor or antagonist of human microRNA-218-5p or the inhibitor or antagonist of human microRNA-23b-3p or a mixture thereof. For example, the pharmaceutical composition may comprise an inhibitor/antagonist of human microRNA-218-5p or an inhibitor/antagonist of human microRNA-23b-3p or mixtures thereof. Preferably, the inhibitor(s)/antagonist(s) present will be present at a concentration that allows the administration of a therapeutically effective dose.

The compositions of the present invention can be aqueous compositions comprising an effective amount of the administration vehicle and comprising either the oligonucleotide or the oligonucleotide analogue in an independent manner or forming liposomes or other complexes, or expression vectors thereof, dissolved or dispersed in a pharmaceutically acceptable vehicle or an aqueous medium. The expressions "pharmaceutically acceptable" or "pharmacologically acceptable" refer to molecular entities and compositions that do not cause adverse reactions, allergic reactions, or reactions of another type, when administered to an animal or a human being. As used herein, "pharmaceutically acceptable vehicle" includes solvents, buffers, solutions, dispersion media, coatings, antibacterial and antifungal agents, isotonic agents, and absorption retardants, and the like acceptable for use thereof in pharmaceutical formulation products, such as pharmaceutical products suitable for administration to human beings.

As shown in the examples, the antagonism of a microRNA which inhibits endogenous MBNL1 protein resulted in a reversal of Fuchs dystrophy phenotype, both when the oligonucleotide was conjugated to oleic acid, and when it is not. Therefore, a **second aspect** of the present invention relates to the use of the oligonucleotide or oligonucleotide analogue as defined in the first aspect or in any of the embodiments comprised in said aspect, in the treatment of Fuchs endothelial corneal dystrophy (FECD).

Preferably, the use is for preparing a drug for the treatment or therapy or prevention of FECD, particularly for mitigating, reducing, alleviating, preventing, or eliminating symptoms of the disease in a patient. The term "treatment" refers to reducing or alleviating symptoms in a subject, preventing the symptoms from worsening or evolving, and/or preventing the disease in a subject who is not sick. Furthermore, the terms "treatment" and "therapy" include preventive and curative methods, since they are both intended for maintaining and/or restoring the health of an individual or animal. Regardless of the origin of the symptoms, the disease, and the incapacity, the administration of a suitable drug to alleviate, prevent, and/or cure a health problem should be interpreted as a way of treatment or therapy in the context of this description.

Therefore, the present invention provides therapeutic and prophylactic methods for the treatment of a subject suffering from FECD or at risk (or susceptible) of suffering from FECD, wherein the method consists of administering one or more of the oligonucleotides or oligonucleotide analogues described in the present invention.

The terms "individual", "patient", or "subject" are used interchangeably herein and do not seek to be limiting in any way. The "individual", "patient", or "subject" can be of any age and sex, and may be in any physical condition. Preferably, the subject is a human patient with FECD. In a preferred embodiment, the treated subject is a human patient having FECD and myotonic muscular dystrophy, preferably myotonic dystrophy type 1 (DM1). Preferably, the subject is a healthy human who is, however, predisposed to or at high risk of having FECD. In a preferred embodiment, the subject is a healthy human who is, however, predisposed to or at high risk of having FECD and myotonic muscular dystrophy, preferably myotonic dystrophy type 1 (DM1). In a preferred embodiment, the subject is a human with myotonic muscular dystrophy, preferably myotonic dystrophy type 1 (DM1), and who suffers from or is predisposed to or at high risk of having FECD.

The use involves the administration of the oligonucleotides or analogues thereof in a therapeutically effective amount. The term "therapeutically effective amount" refers to an amount of oligonucleotides or analogues thereof which has a therapeutic effect and is capable of treating FECD.

The use may involve the administration of the oligonucleotides or analogues thereof, for example, by the subcutaneous or systemic route, preferably by the intravenous route, for example dissolved or suspended in a pharmaceutically acceptable vehicle, such as water or an aqueous solution such as, for example, saline or phosphate buffer. The composition in which they are administered may contain pharmaceutically acceptable excipients. Furthermore, the composition may contain particles, preferably nanoparticles, more preferably lipid nanoparticles, carrying the oligonucleotides or analogues thereof.

The use also includes administration through the oral, nasal, ocular, intraocular, or buccal route, and preferably administration can also be through the intradermal, subcutaneous, intramuscular, intraperitoneal, or intravenous route. As discussed above, the compositions comprising antagomiRs or antimiRs are preferably formulated for intravenous or subcutaneous administration. Preferably, the administration is local administration, preferably in the eye. More preferably, the administration is in the eye, and the oligonucleotides or analogues thereof as defined in the first aspect are conjugated to, or carried in, particles, preferably lipid nanoparticles.

In a third aspect, the present invention relates to a kit, wherein the kit comprises the oligonucleotides defined in the first aspect, or any of the preferred embodiments thereof. Said kit can further include other compounds, for example for the administration of the oligonucleotides, or instructions of use.

### SEQUENCE LISTING

The following nomenclature is used throughout the entire specification to define the chemical modifications included in the sequences disclosed herein:
- Locked nucleic acids or LNAs are represented by means of the combination of a capital letter and "b" in lower case: Ab, Gb, Tb, Cb.
- Phosphorothioate linkages are indicated with "s" in lower case.
- 2'-O-methoxyethyl (MOE) nucleotides are represented by means of the combination of a capital letter and "m" in lower case: Am, Cm, Gm, Tm.
- 2'-O-methyl-nucleotides are represented with letters of the nucleobase in lower case: a, g, c, u.
- 5-Methyl-2'-O-methylcytidine nucleotides are represented by means of the expression (5Mc).
- The expression "(AO)" indicates that the oligonucleotide or its analogue is conjugated to an oleic acid molecule.
- The expression "spacer" indicates that there is a connector between the oligonucleotide and oleic acid, wherein the spacer is preferably selected from the group of NHC3, NHC5, NHC6, threoninol, or derivatives thereof.
- "Y" means any pyrimidine (C or U/T).
- "I" means hypoxanthine.

**SEQ ID NO: 1:** hsa-miR-218-5p: U**UGUGCU**UGAUCUAACCAUGU
**SEQ ID NO: 2:** hsa-miR-23b-3p: A**UCACAU**UGCCAGGGAUUACCAC
**SEQ ID NO: 3:** seed region of hsa-miR-218-5p**:** UGUGCU
**SEQ ID NO: 4:** seed region of hsa-miR-23b-3p: UCACAU
**SEQ ID NO 5**: Antagonist of human hsa-miR-218-5p: TTAGATCAAGCACAA
**SEQ ID NO 6**: Antagonist of human hsa-miR-23b-3p: ATCCCTGGCAATGTGA
**SEQ ID NO: 7:** Antagonist of hsa-miR-23b-3p, X82102: AbsTbs(5Mc)s(5Mc)sCmTbGmsgsCmsAbAmTbGbTmsGbsAb
**SEQ ID NO: 8:** Antagonist of hsa-miR-23b-3p, X82108: AbsTbs(5Mc)s(5Mc)sCmTbGmsgsCmsAbAmTbGbTmsGbsAb(OA), wherein OA can be bound to the oligonucleotide directly or through a spacer, wherein the spacer is preferably selected from the group of NHC3, NHC5, NHC6, threoninol, or derivatives thereof.
**SEQ ID NO 9**: Antagonist of hsa-miR-23b-3p, X82108: AbsTbs(5Mc)s(5Mc)sCmTbGmsgsCmsAbAmTbGbTmsGbsAb(NHC6)(OA), wherein OA is bound to the oligonucleotide through a spacer which is NHC6.
**SEQ ID NO 10**: Antagonist of human hsa-miR-218-5p, 218 MOE (X82109): TbsTbsAmsGbsAmsTmsCbAmsAmGbCmAbsCmsAbsAb
**SEQ ID NO 11**: Antagonist of human hsa-miR-218-5p, 218 MOE Oleic 3': TbsTbsAmsGbsAmsTmsCbAmsAmGbCmAbsCmsAbsAb(OA), wherein OA can be bound to the oligonucleotide directly or through a spacer, wherein the spacer is preferably selected from the group of NHC3, NHC5, NHC6, threoninol, or derivatives thereof.
**SEQ ID NO 12**: Antagonist of human hsa-miR-218-5p, 218 MOE Oleic 3': TbsTbsAmsGbsAmsTmsCbAmsAmGbCmAbsCmsAbsAb(NHC6)(OA), wherein OA is bound to the oligonucleotide through a spacer which is NHC6.
**SEQ ID NO 13**: Antagonist of human hsa-miR-23b-3p, MD23b-2-PS/PO: AbsTms(5Mc)s(5Mc)(5Mc)Tbgsgs(5Mc)sAbAmTbGbsTmsGbsAb
**SEQ ID NO 14**: Antagonist of human hsa-miR-23b-3p, MD23b-2-PS/PO 3'OI: AbsTms(5Mc)s(5Mc)(5Mc)Tbgsgs(5Mc)sAbAmTbGbsTmsGbsAb(OA), wherein OA can be bound to the oligonucleotide directly or through a spacer, wherein the spacer is preferably selected from the group of NHC3, NHC5, NHC6, threoninol, or derivatives thereof.
**SEQ ID NO 15**: Antagonist of human hsa-miR-23b-3p, MD23b-2-PS/PO 3'OI: AbsTms(5Mc)s(5Mc)(5Mc)Tbgsgs(5Mc)sAbAmTbGbsTmsGbsAb(NHC6)(OleicAcid) (NHC6)(OA), wherein OA is bound to the oligonucleotide through a spacer which is NHC6.

The invention will not be illustrated in more detail with the help of the examples and figures shown below.

### EXAMPLES

### EXAMPLE 1: Effectiveness of the antagonists against microRNA-23b.

### Methodology

### In vitro model - Primary corneal endothelial cell culture

Corneal cells were obtained from control donors and cornea-transplanted donors diagnosed with FECD. These cells were first disaggregated with collagenase (Life Technologies, #17018-029) at 37°C for 3 hours. The cells were then centrifuged at 1000 G for 3 min at RT and trypsinised with TrypLE select (Life Technologies, #12563-029) at 37°C for 5 min. Finally, they were centrifuged again and resuspended in endothelial SFM medium supplemented with FBS (Biowest, #51810-500), Y27632 inhibitor (Miltenyi Biotec, #130-104-169), penicillin/streptomycin, and fungizone (Biowest, #L0009-100). Finally, the resulting pellet was seeded in a Millicell EZ 8-well glass slide. The medium was changed to Ham 12 and M199 (Merck Life Science, #N6658 and #M4530) supplemented with FBS, Y27632, FGF, insulin (Thermo Fisher Scientific, #13256-029), ascorbic acid (Sigma Aldrich, #49752-10G), penicillin/streptomycin, and amphotericin B every 48 hours. Upon reaching 80% confluence, medium is changed to endothelial SFM medium. Lastly, passage for the final culture and treatment with nanoparticles was performed with a trypsinisation protocol using Triple select, inactivation with FBS, and centrifugation at 150 G for 5 min at room temperature.

### In vitro treatment of models

Primary corneal endothelial cells from healthy donors were treated with oligonucleotides X82102 (naked), X82108 (ATX-01), and X82108-Cy3 at a concentration of 200 nM for 96 hours. Treatment with sterile PBS was also performed as control.

Similarly, primary corneal endothelial cells from FECD patients were treated with PBS, X82108, and X82108-Cy3, under the same conditions mentioned above.

Upon treatment completion, the cells were fixed with 4% paraformaldehyde (Thermo Fisher Scientific, #043368-9M) for fluorescence methods.

### In vitro fluorescence methods in models

Endothelial markers were used to describe the model. A double ZO-1 and PDRX-6 staining was performed on primary control cells and primary cells from patients. Corneal cells were fixed in 4% PFA and permeabilised in PBS. A blocking solution, with donkey serum and bovine serum albumin, was used to prevent non-specific binding. Primary anti-ZO1 antibodies (1:200, Invitrogen #40-2200) and anti-PDRX6 antibodies (1:200, Invitrogen #MA5-34906), and secondary Alexa 594-conjugated anti-rabbit antibodies (1:200, Invitrogen #A32754) and Alexa 488-conjugated anti-mouse antibodies (1:200, Invitrogen #A32723) were used, respectively.

The successful entry of the oligonucleotide into the models *in vitro* was studied by means of fluorescence methods. Cy3-conjugated oligonucleotide was visualised, so immunofluorescence staining is not required.

MBNL1 protein expression was also studied in order to study the effects of the drug on this direct miR-23b target involved in the pathology. Corneal cells were fixed in 4% PFA and permeabilised in PBS. A blocking solution, with donkey serum and bovine serum albumin, was used to prevent non-specific binding. An anti-MBNL1 antibody (1:200, MB1a (4A8), MDA Monoclonal Antibody Resource) and an Alexa 488-conjugated anti-mouse antibody (1:200, Invitrogen #A32723) were used for detecting the protein.

Fluorescen*t in situ* hybridisation was then performed to detect the RNA foci carrying CUG repeats. Cells were incubated in a hybridisation buffer (40% formamide, 2x SSC, 0.2% BSA, 10% dextran sulphate, 2 mM ribonucleoside-vanadyl complex, 10% tRNA [10 mg/ml], and 10% herring sperm) for 30 minutes at room temperature and hybridised with a Cy3-(CAG)7-Cy3-labelled probe diluted to 1:500 in the hybridisation buffer for 2 h at 37°C in the dark. After hybridisation, 3 washes were performed with 2x SSC + 30% formamide for 10 minutes at 37°C, then 3 washes were performed with 0.5x SSC for 10 minutes at 37°C, and one wash was performed for 10 minutes at room temperature, and lastly 3 washes were performed with sterile PBS for 5 minutes at room temperature.

Lastly, all the preparations were mounted with Vectashield^{®} plus Antifade Mounting Medium with DAPI (Vector Laboratories, #H-2000) to view the nuclei. The preparations were viewed under a confocal microscope.

### Image analysis

Image J Fiji App was used for image analysis. CUG nuclear foci were quantified by means of a macro counting the number of nuclear foci with a certain circularity in the red channel which co-localised with the nuclei of corneal cells (stained with DAPI). These were normalised to the number of total nuclei. Nuclear MBNL1 aggregates were counted manually and likewise normalised to the number of total nuclei. MBNL1 intensity was analysed by means of a macro measuring the integrated density of the green channel in the area occupied by the cells, and this was then relativised to PBS values.

### Statistical analysis

Statistical analysis was performed by means of the GraphPad Prism 8 statistic programme. Said programme allowed performing statistical analyses and graphical representation. The Shapiro-Wilk test of normality was applied before statistical analysis, once it was verified that the data was normal, one-way ANOVA analyses were used for multiple comparisons between more than 2 groups and a one-tailed t test was used for comparisons between 2 groups.

### Results

The results obtained demonstrate that the oligonucleotide conjugated to oleic acid (X82108) is not only capable of penetrating primary corneal endothelial cells (Figure 3), but also increases MBNL1 levels (Figure 4). Similarly, the oligonucleotide not conjugated to oleic acid (X82102) can enter corneal cells and increase MBNL1 levels (Figure 4). Furthermore, it was observed that miR-23b-inhibiting oligonucleotide X82108 can restore molecular markers in the primary cells from patients with FECD and lead them to a healthy phenotype (Figure 5), since it did not only increase the density of MBNL1, but also reduced RNA foci and nuclear MBNL1 aggregates.

Therefore, it is concluded that the microRNA inhibitors which inhibit MBNL, particularly microRNA-23b, are potential drugs for treating patients diagnosed with FECD.

### EXAMPLE 2: Effectiveness of the antagonists against microRNA-218

### Primary corneal endothelial cell culture

Corneal endothelial cells were obtained from cornea-transplanted control donors. Corneal epithelium was isolated and disaggregated using collagenase (Life Technologies, #17018-029) at 37°C for 3 hours. The cells were then centrifuged at 1000 G for 3 minutes at room temperature and subjected to trypsinisation with TrypLE Select (Life Technologies, #12563-029) at 37°C for 5 minutes. Thereafter, they were centrifuged again and resuspended in endothelial SFM medium supplemented with FBS, Y27632 inhibitor (Miltenyi Biotec, #130-104-169), penicillin/streptomycin, and fungizone (Biowest, #L0009-100). The cells obtained were seeded in a Millicell EZ 8-well glass slide. Every 48 hours, the culture medium was replaced with a mixture of Ham 12 and M199 (Merck Life Science, #N6658 and #M4530) supplemented with FBS, Y27632, FGF, insulin (Thermo Fisher Scientific), ascorbic acid (Sigma Aldrich, #49752-10G), penicillin/streptomycin and amphotericin B. Upon reaching 80% cell confluence, the medium was replaced with endothelial SFM. Lastly, for passage to final culture and treatment with the antimiR X35004, a new trypsinisation was performed, the cells were inactivated with FBS and centrifuged at 150 G for 5 minutes at room temperature.

### Treatment with AntimiRs

Primary corneal endothelial cells obtained from healthy donors were treated with the antimiR X82109 (AntimiR-218) at a concentration of 200 nM for 96 hours. Sterile PBS was used as control. After treatment, the cells were fixed with 4% paraformaldehyde (Thermo Fisher Scientific, #043368-9M) for subsequent analysis by means of immunofluorescence.

### Immunofluorescence of MBNL1

The expression of the MBNL1 protein, a known target of miR-218, was measured to analyse the effects of the antimiRs on this protein involved in the pathology. Corneal endothelial cells were fixed with 4% paraformaldehyde and permeabilised in PBS. A blocking solution containing donkey serum and bovine serum albumin was used to prevent non-specific binding. MBNL1 detection was performed using a primary anti-MBNL1 antibody (1:200, MB1a (4A8), MDA Monoclonal Antibody Resource) and a secondary Alexa 488-conjugated anti-mouse antibody (1:200, Invitrogen, #A32723). Lastly, the samples were mounted with Vectashield^{®} Plus Antifade Mounting Medium with DAPI (Vector Laboratories, #H-2000) to view the nuclei, and they were observed under a ZEISS Axio Imager Z2 confocal microscope.

Image analysis was performed using the ImageJ Fiji application. The intensity of the MBNL1 protein was evaluated by means of a macro calculating the integrated density of the green channel in the areas corresponding to the cells. The obtained values were then normalised in relation to the values of the PBS-treated control.

### Statistical analysis

Statistical analysis was performed using the GraphPad Prism 10 software, facilitating both the analysis and the graphical representation of data. The Shapiro-Wilk test of normality was applied before analysis to verify the normal distribution of the data. When normality has been confirmed, a one-tailed t test was used for statistical comparison.

### Results

The results obtained in this example confirm that, as can be seen in Figure 6, microRNA inhibitors which inhibit MBNL (for example, miR-218) are potential drugs for treating patients diagnosed with FECD.

## Claims

1. An oligonucleotide or oligonucleotide analogue for use thereof in the treatment or prevention of Fuchs endothelial corneal dystrophy (FECD), wherein the oligonucleotide or oligonucleotide analogue is a microRNA antagonist that down regulates the expression of the human gene MBNL1 and/or MBNL2, wherein the microRNA that down regulates the expression of the human gene MBNL1 and/or MBNL2 is human microRNA-23b-3p or human microRNA-218-5p.

2. The oligonucleotide or oligonucleotide analogue for use thereof according to claim 1, wherein the oligonucleotide or oligonucleotide analogue is an antimiR comprising a nucleobase sequence which is 100% complementary to the nucleobase sequence of the seed region of human microRNA-23b-3p defined as SEQ ID NO: 4, or 100% complementary to the nucleobase sequence of the seed region of human microRNA-218-5p defined as SEQ ID NO: 3.

3. The oligonucleotide or oligonucleotide analogue for use thereof according to any of claims 1 or 2, wherein the oligonucleotide or oligonucleotide analogue comprises a nucleobase sequence which is 100% complementary to the nucleobase sequence of SEQ ID NO: 2 (human microRNA-23b-3p) or SEQ ID NO: 1 (human microRNA-218-5p).

4. The oligonucleotide or oligonucleotide analogue for use thereof according to any of claims 1 to 3, wherein the oligonucleotide or oligonucleotide analogue comprises the nucleobase sequence of SEQ ID NO: 6 or SEQ ID NO: 5, or a sequence that is at least 85% identical to the nucleobase sequence of SEQ ID NO: 6 or SEQ ID NO: 5, respectively.

5. The oligonucleotide or oligonucleotide analogue for use thereof according to any of claims 1 to 4, wherein the oligonucleotide is an oligonucleotide analogue and:
a) at least one of the nucleotides making up same has one or more chemical modifications in the ribose moiety, phosphate linkage, or both,
b) comprises a nucleobase sequence which is 100% complementary to the nucleobase sequence of the seed region of the microRNA of SEQ ID NO: 1 (human microRNA-218-5p) or SEQ ID NO: 2 (human microRNA-23b-3p), and
c) optionally has a conjugated fatty acid at the 5' end and/or the 3' end.

6. The oligonucleotide or oligonucleotide analogue for use thereof according to claim 5, wherein the oligonucleotide analogue comprises the nucleobase sequence and chemical modifications of SEQ ID NOs: 7, 13, or 10.

7. The oligonucleotide or oligonucleotide analogue for use thereof according to any of claims 5 or 6, wherein the oligonucleotide analogue has an oleic acid conjugated at its 3' end.

8. The oligonucleotide or oligonucleotide analogue for use thereof according to claim 7, wherein the oleic acid is conjugated to the oligonucleotide analogue through a spacer molecule, preferably NHC6.

9. The oligonucleotide or oligonucleotide analogue for use thereof according to claim 8, wherein the oligonucleotide analogue consists of SEQ ID NO: 8 or 9.

10. The oligonucleotide or oligonucleotide analogue for use thereof according to claim 8, wherein the oligonucleotide analogue consists of SEQ ID NO: 14 or 15.

11. The oligonucleotide or oligonucleotide analogue for use thereof according to claim 8, wherein the oligonucleotide analogue consists of SEQ ID NO: 11 or 12.

12. The oligonucleotide or oligonucleotide analogue for use thereof according to any of claims 1 to 11, wherein said oligonucleotide or oligonucleotide analogue is administered to a subject suffering from FECD or at risk of suffering from FECD, and furthermore suffering or at risk of suffering from myotonic dystrophy type 1.
